# EUROPÄISCHE PATENTSCHRIFT

(11) **EP 3 240 531 B1**
(45) Veröffentlichungstag und Bekanntmachung des Hinweises auf die Patenterteilung: **12.02.2020**
(21) Anmeldenummer: 15837208.6
(22) Anmeldetag: 28.12.2015
(51) Int. Cl.: A61K 9/14, A61K 33/26, A61K 9/00

(54) **ARZNEIMITTEL AUF DER BASIS VON MAGHÄMIT ZUR GLEICHZEITIGEN REDUZIERUNG DER GASTROINTESTINALEN NATRIUMRESORPTION UND PHOSPHATRESORPTION**
DRUG BASED ON MAGHEMITE FOR SIMULTANEOUS REDUCTION OF GASTROINTESTINAL SODIUM RESORPTION AND PHOSPHATE RESORPTION
MÉDICAMENTS À BASE DE MAGHÉMITE POUR LA RÉDUCTION SIMULTANÉE DE L'ABSORPTION DU SODIUM ET DU PHOSPHATE DANS LE TRACTUS GASTRO-INTESTINAL

(30) Priorität: 29.12.2014 DE 102014019388
(43) Veröffentlichungstag der Anmeldung: 08.11.2017
(73) Patentinhaber: Charité - Universitätsmedizin Berlin, 10117 Berlin (DE)
(72) Erfinder: TAUPITZ, Matthias, 15831 Mahlow (DE); SCHNORR, Jörg, 16515 Oranienburg / OT Lehnitz (DE); EBER, Monika, 15831 Blankenfelde-Mahlow / OT Mahlow (DE); STOLZENBURG, Nicola, 14165 Berlin (DE); GLÄSER, Janna, 10557 Berlin (DE); KRATZ, Harald, 10247 Berlin (DE); HAUPTMANN, Ralf, 12203 Berlin (DE); BREINL, Janni, 14167 Berlin (DE); ARIZA DE SCHELLENBERGER, Angela, 10115 Berlin (DE); GEMEINHARDT, Ines, 13509 Berlin (DE); WAGNER, Susanne, 15831 Blankenfelde-Mahlow/ OT Mahlow (DE)
(74) Vertreter: Gulde & Partner
(86) Internationale Anmeldenummer: PCT/DE2015/000608
(87) Internationale Veröffentlichungsnummer: WO 2016/107619

(56) Entgegenhaltungen:
- WO-A1-2013/034267
- T.M.-H. NGUYEN ET AL: "Novel oral phosphate binder with nanocrystalline maghemite-phosphate binding capacity and pH effect", INTERNATIONAL JOURNAL OF PHARMACEUTICS, Bd. 482, Nr. 1-2, 6. November 2014 (2014-11-06), Seiten 21-26, XP55264761, NL ISSN: 0378-5173, DOI: 10.1016/j.ijpharm.2014.11.007
- H. VETHE ET AL: "EXPERIMENTAL PATHOLOGY", NEPHROLOGY DIALYSIS TRANSPLANTATION., Bd. 29, Nr. suppl 3, 1. Mai 2014 (2014-05-01), Seiten iii201-iii208, XP55264750, GB ISSN: 0931-0509, DOI: 10.1093/ndt/gfu152

## Beschreibung

Die vorliegende Erfindung bezieht sich auf ein Verfahren zur Herstellung von Maghämit-Kristallen.

### Hintergrund der Erfindung

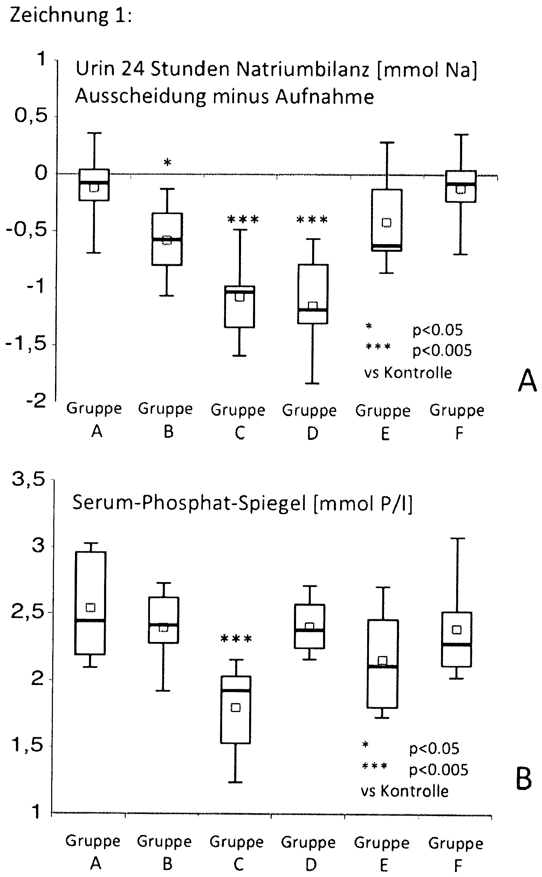

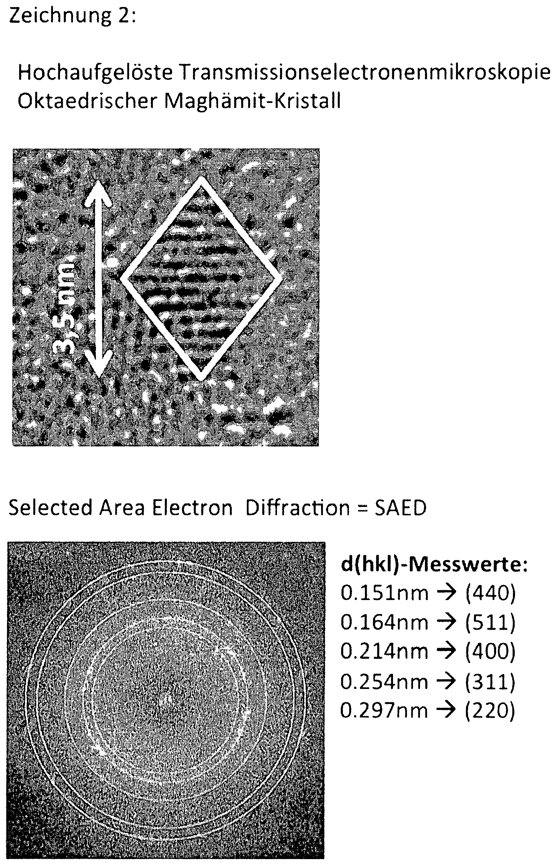

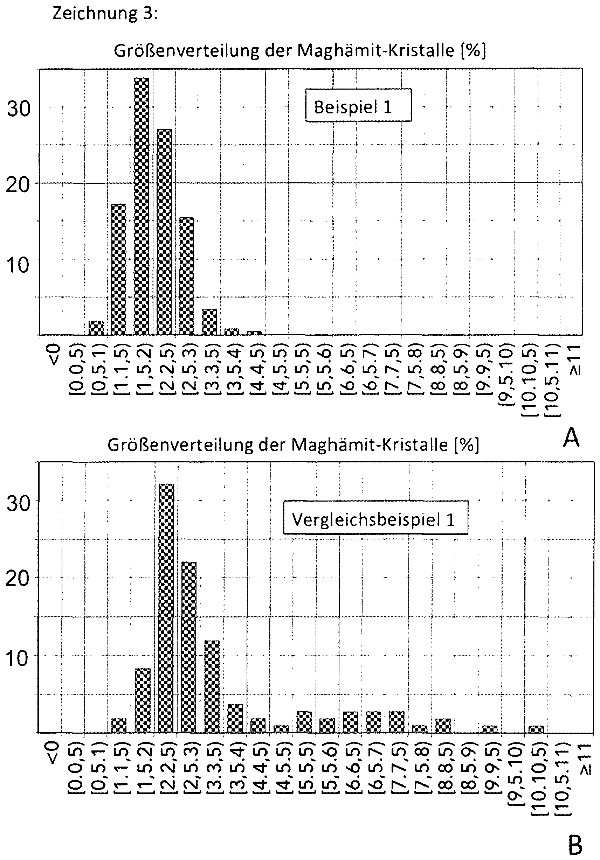

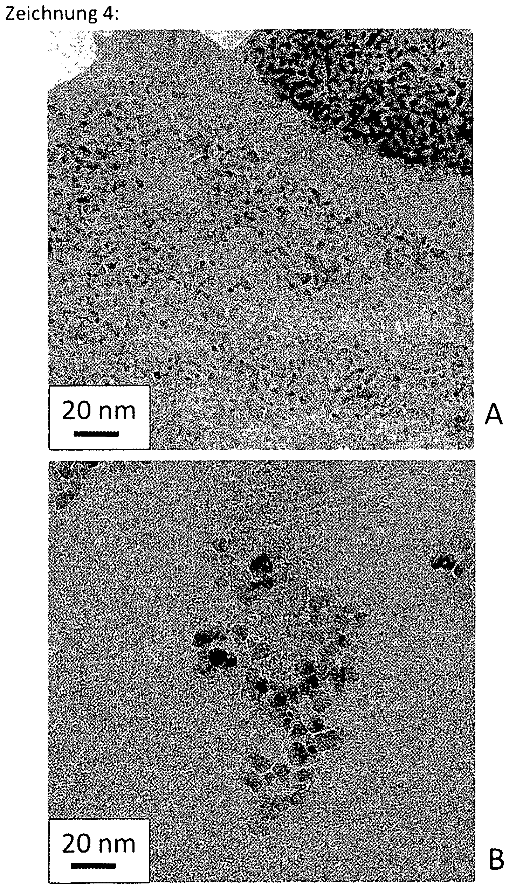

Patienten mit eingeschränkter Nierenfunktion weisen leicht eine ungünstige Natriumbilanz auf und haben eine Hyperphosphatämie, da die Niere das Natrium und das Phosphat nicht mehr in ausreichendem Maß ausscheiden kann. Zudem kommt es zu Imbalanzen in der hormonellen Steuerung des Mineral- und Elektrolythaushaltes. Natriumaufnahme im Darm führt sekundär zu Wassereinlagerungen in die Körpergewebe, zu einem höheren Blutvolumen und zu Bluthochdruck, was die Nierenerkrankung verschlimmert. Ein zu hoher Serumphosphatspiegel führt zu atherosklerotischen Gefäßwandveränderungen mit Erhöhung des Risikos für kardiovaskuläre Ereignisse, wie Schlaganfall oder Herzinfarkt. Neue Entwicklungen zielen auf Stoffe, die die Natriumaufnahme im Darm oder Phosphataufnahme im Darm hemmen. Ein Stoff, der den NHE (Na⁺/H⁺Exchange)-Transportmechanismus, wie in US 2012/0263670 dargelegt, hemmt, führt an gesunden Ratten zu einer signifikanten Verminderung der Natriumresorption, was sich in der Reduzierung der Natriumausscheidung im Harn dokumentiert. Gemäß Labonte et al. (2014, Journal of American Society of Nephrology 26, online Publikation doi: 10.1681/ASN.2014030317) bewirkt diese Stoffgruppe in ersten präklinischen in-vivo Studien an Ratten in einem Wirkstoff-Ko-Futter-Mischversuch an gesunden Tieren eine Reduktion der Natriumausscheidung durch verminderte Natriumaufnahme um 0,3 mmol im Vergleich zu einer Kontrollgruppe und eine Reduktion der Phosphatausscheidung (vergleiche Abbildung 4A und 4B in Labonte et al.). Dieses Arzneimittel bewirkt zudem in einer Studie an gesunden Ratten eine minimale Reduzierung der Phosphatresorption im Darm und damit eine Reduzierung der Urinausscheidung, jedoch keine signifikante Minderung des Serumphosphatspiegels. Gemäß Labonte et al. führt eine Erhöhung der Dosis oder die Verwendung eines Stoffes mit stärkerer Wirkung zu starken Durchfällen. Ein Stoff, der gleichzeitig signifikant den Serumphosphatspiegel durch Hemmung der gastrointestinalen Resorption senken kann und die Natriumresorption hemmen kann und dies nicht mit erhöhtem Stuhlvolumen und Durchfall einhergeht, ist nicht bekannt.

### Beschreibung der Erfindung

Die erfindungsgemäße Verfahren zur Herstellung von Maghämit-Kristallen mit einer Kristallgröße im Bereich von 0.5 bis 4 nm und einem Magnetitanteil von kleiner als 5 Gewichtsanteilen von Hundert, wobei sich der Gewichtsanteil auf den Anteil zweiwertiger Eisenionen am Gesamteisen bezieht, umfasst die Schritt:
- S1:: Herstellen einer wässrigen Natriumhydroxid-Lösung mit einem pH-Wert im Bereich von 10 bis 15, wobei die Natriumhydroxid-Lösung ein oder mehrere Alditole sowie ein oder mehrere Kohlenhydrate enthält und die Temperatur der Natriumhydroxid-Lösung 0 bis 10° Celsius beträgt;
- S2:: Herstellen einer weiteren wässrigen Lösung, die Chlorid-Ionen, Eisen-II-Salze und Eisen-III-Salze enthält und eine Temperatur von 0 bis 10° Celsius aufweist; und
- S3:: Herstellen einer Mischung aus der Natriumhydroxid-Lösung und der weiteren Lösung, wobei die Temperatur der Mischung 0 bis 10° Celsius beträgt.

Überraschenderweise führt ein Stoff bestehend aus Maghämit mit einer Herstellung gemäß Beispiel 1 und Hilfsstoffen aus Mannitol, Inulin und Gummi Arabicum bei oraler Verabreichung zu einer Reduktion der gastrointestinalen Resorption von Natrium und zu einer verminderten Ausscheidung über den Urin an gesunden Ratten, wie in Beispiel 2 dargestellt. Gleichzeitig führt der Stoff gemäß Beispiel 1 in der gemäß Beispiel 2 getesteten Dosis zu einer Verminderung des Serumphosphatspiegels, ohne Nebenwirkungen wie Durchfall. Diese Reduktion der Natriumresorption ist überraschenderweise mit einer Reduktion der gastrointestinalen Phosphatresorption verbunden und führt gemäß Beispiel 2 zu einer Hypophosphatämie, die sich durch eine reine chemische adsorptive Wirkung des Phosphates an das Eisenoxid nicht erklären lässt. Das Ausmaß der gemäß Beispiel 2 mit dem Stoff gemäß Beispiel 1 erreichten Hypophosphatämie übertrifft bei Weitem das Maß, wie es durch reine chemische adsorptive Wirkung des Eisenoxides in Form von Maghämit im erfindungsgemäß hergestelltem Stoff gegenüber dem Phosphat in der Nahrung erklärbar wäre. Der Futtergehalt an Phosphor ist 0,7 % (Gewicht), kann somit theoretisch in einer Menge von 2,1 % Phosphat (Gewicht) zur Verfügung stehen. Bei einer Zugabe des Stoffes gemäß Beispiel 1 bezogen auf Gewichtsanteile Eisen als Bestandteil des Maghämit von 0.25 % kann selbst bei optimaler Phosphatbindung nicht dazu führen, dass dem Organismus nicht genug freies Phosphat zu gastrointestinalen Aufnahme zur Verfügung steht. Im Vergleich zu Labonte et al. ist die Wirkung des erfindungsgemäßen Stoffes basierend auf Maghämit auf die Natriumresorption durch Beispiel 1 dreimal höher (Reduktion der Natriumausscheidung um 1 mmol) als ein spezifisch hierfür entwickelter Inhibitor gemäß US 2012/0263670. Für den Stoff gemäß Beispiel 1 erfolgt diese Reduktion der Natriumresorption und renalen Ausscheidung ohne Nebenwirkungen wie Durchfall, so wie es für den Stoff gemäß US 2012/0263670 und Labonte et al. beschrieben ist. So ergibt sich für den Stoff gemäß Beispiel 1 eine sehr hohe therapeutische Breite im Gegensatz zu dem Stoff gemäß US 2012/0263670 und Labonte et al.. In den in-vivo Studien gemäß Beispiel 2 zeigt sich, dass das Urinvolumen nicht signifikant reduziert wird und das Stuhlgewicht nicht signifikant erhöht wird. Dies zeigt, dass die hier gefundene Wirkung nicht mit Nebenwirkungen einhergeht.

Der in Beispiel 1 dargestellte Stoff wird durch gleichzeitige Herstellung und Kristallbildung in Anwesenheit eines Alditols und eines Fructans definiert. Der Stoff wird daher primär durch das Fructan, hier Inulin, bestimmt. In einer simulierten gastrointestinalen Passage zeichnet der Stoff gemäß Beispiel 1 gegenüber Vergleichsbeispiel 1 dadurch aus, dass bei einem Überschuss von anorganischem Phosphat in der Inkubationslösung die Phosphatbindungskapazität um 60% höher liegt. Hiermit ist belegt, dass die primäre Inkubation der Alditole und Kohlenhydrate bei einem pH-Wert über 11 vor der Mischung mit den Eisensalzen zu einer chemischen Veränderung der Alditole und Kohlenhydrate führt, welche zu einem Stoff führt, der sich von dem Stoff gemäß Vergleichsbeispiel 1 unterscheidet. Darüber hinaus zeichnet sich der Stoff gemäß Beispiel 1 überraschenderweise durch eine höhere Stabilität aus, die dadurch deutlich wird, dass der Anteil an freiem Eisen geringer ist in der gastrointestinalen Passage als für den Stoff gemäß Vergleichsbeispiel 1. Dieser Einfluss auf die renale Natriumausscheidung durch den Stoff gemäß Beispiel 1 geht nicht mit einer Senkung des Serumnatriumspiegels einher. Das Gesamtgewicht des Kots unterscheidet sich nicht von den Vergleichsgruppen. Ebenso wenig hat der erfindungsgemäße Stoff nach Beispiel 1 keinen Einfluss auf die Kaliumausscheidung und Proteinausscheidung und auf das Urinvolumen in dem 24 Stunden Sammelversuch. Das unter dem Namen Velphoro® erhältliche Arzneimittel mit dem Wirkstoff Sucroferric Oxyhydroxide ist vergleichbar dem hier dargestellten Stoff gemäß Beispiel 1 eine spezielle Form des Eisenoxides mit Hilfsstoffen aus der Gruppe der Kohlenhydrate. Der Wirkstoff Velphoro® ist in WO 97/22266 A1 beschrieben. Velphoro® bewirkt eine signifikante Reduktion der gastrointestinalen Natriumresorption, zeigt jedoch in der hier untersuchten Dosis keine Wirkung auf den Serumphosphatspiegel. Dies belegt, dass eine derartig starke Wirkung auf die gastrointestinale Phosphatresorption nicht mit jeder Art von Eisenoxid in Verbindung mit einem Kohlenhydrat erreicht werden kann. Der Stoff gemäß Beispiel 1 ist Velphoro® aufgrund der kombinierten Reduzierung der Phosphatresorption und der gleichzeitigen Reduzierung der Natriumresorption überlegen. Vermutlich senkt der Stoff gemäß Beispiel 1 selektiv die Aufnahme von Natrium und Phosphat aus dem Darm, was sich in einer hochsignifikanten Reduktion der Ausscheidung des Natriums über die Niere und in einer hochsignifikanten Senkung des Serumphosphatspiegels wiederspiegelt. Der Stoff gemäß Beispiel 1 ist dahingehend nicht nur den Arzneimitteln Velphoro®, Renvela® und Fosrenol® überlegen, sondern auch neuen speziellen Transport-Inhibitoren wie denen aus US 2012/0263670 und der Veröffentlichung von Labonte et al., sowie den in WO 2012/0006475 A1 beschriebenen Stoffen. Die Wirkung des Stoffes gemäß Beispiel 1 auf die gastrointestinale Phosphataufnahme ist so stark, dass die gesunden Ratten in eine Hypophosphatämie fallen, was zu einem signifikant geringeren Gewicht der Ratten am Ende des Versuches führte. Diese Nebenwirkung kann leicht durch Dosisreduktion behoben. Es wurde festgestellt, dass allein die Kombination der hier verwendeten Hilfsstoffe im Vergleich zur Kontrollgruppe einen signifikanten Einfluss auf die renale Natriumausscheidung aufweist. Der erfindungsgemäße Stoff wird in einer nasschemischen wässrigen über die primäre Fällung von Magnetit in Anwesenheit von Eisen-(II)-Chlorid und Eisen-(III)-Chlorid bei Temperaturen unter 10° Celsius und in Anwesenheit von Mannitol und Inulin hergestellt. Nach primärer Kristallbildung wird das kristalline Magnetit aktiv zu Maghämit oxidiert mittels Wasserstoffperoxid bei Temperaturen über 50° Celsius und abschließend werden die nicht umgesetzten Ausgangsstoffe und unerwünschten Reaktionsprodukte mittel Dialyse, Diafiltration oder auch Ultrafiltration entfernt. Denkbar sind zudem Mischungen aus anderen Alditolen, monomeren Hexosen, monomeren Pentosen sowie deren Polymere.

Erfindungsgemäß bewirkt der Stoff eine Reduktion der Natriumaufnahme im Darm, die mit einer signifikanten Reduktion der Ausscheidung von Natrium im Urin einhergeht, ohne Einfluss auf den Serum-Natriumspiegel zu haben. Überraschenderweise führt der Stoff in der hier getesteten Dosis auch zu einer Reduktion des Serumphosphatspiegels, der durch eine reine adsorptive Wirkung nicht erklärbar ist, da bei einem Futterphosphatgehalt von 2,1% eine Kofütterung des Stoffes nach Beispiel 1 eine Zugabe von 0,25% Eisen bedeutet und daher der Ratte noch ausreichend Nahrungsphosphat für einen ausgeglichenen Phosphat-Stoffwechsel verbliebe. Es wird vermutet, dass eine Verminderung der Natriumresorption im Darm auch einen Einfluss auf die Phosphatresorption hat. Somit zeichnet sich der Stoff gemäß Beispiel 1 auch dadurch aus, dass er eine signifikante Reduktion der gastrointestinalen Natriumresorption durch verminderte renale Natriumausscheidung bei nicht verändertem Serum-Natriumspiegel bewirkt, wobei dies gleichzeitig mit einer signifikanten Reduktion des Serum-Phosphatspiegels einhergeht. Der Effekt auf den Serum-Phopsphatspiegel wird nicht einmal für einen selektiven Hemmstoff der gastrointestinalen Phosphattransporter gefunden, wie WO 2012/0006475 A1, Beispiel 57 auf Seite 758, beschrieben ist. So zeichnet sich der hier erfindungsgemäß dargestellte Stoff durch eine Hemmung von Elektrolyttransportvorgängen und Mineraltransportvorgängen, hier Natrium und Phosphat, in der Darmwand aus, die bisherigen in der Entwicklung befindlichen und zugelassenen Stoffen überlegen ist. Dies kann zur Regulierung der Natrium-, und Wasser- und Phosphatbilanz an Patienten mit eingeschränkter Nierenfunktion bei peroraler Verabreichung in pharmazeutisch bekannten Darreichungsformen ausgenutzt werden. Neben den hier dargestellten Hilfsstoffen aus der Gruppe der Alditole und Kohlenhydrate kann die Herstellung und Anwendung der Maghämitbasierten Nanokristalle zur Reduktion der gastrointestinalen Natrium- und Phosphatresorption mit gängigen bekannten pharmazeutischen Hilfsstoffen und auch Wirkstoffen erfolgen.

Neben dem hier erfindungsgemäß dargestellten Stoff gemäß Beispiel 1 können die Kristalle neben Eisen auch andere Metalle, Metallhydroxide und Metalloxyhydroxide enthalten. Eine Anwendung des Stoffe soll bei oraler Anwendung dazu betragen die gastrointestinale Natriumresorption und gleichzeitig die Phosphatresorption zu vermindern und so an Patienten mit eingeschränkter Nierenfunktion zu einer Verbesserung des Wasser- und Elektrolythaushaltes führen, was sekundär zu einer Senkung des Blutdruckes und der Gefäßwandverkalkung führt und damit das Risiko für kardiovaskuläre Krankheiten wie Schlaganfall und Herzinfarkt bei Patienten mit eingeschränkter Nierenfunktion verbessert.

### Beschreibung der Zeichnungen

### Beschreibung Zeichnung 1 :

Es ist das Ergebnis der in-vivo Untersuchungen gemäß Beispiel 2 an gesunden Ratten dargestellt. Wirkstoffe wurden dem Futter zugemischt und es wurde in einem Stoffwechselkäfig 24 Stunden lang Urin gesammelt und es wurde den Tieren Blut abgenommen. Zeichnung 1A zeigt die Natriumbilanz berechnet aus Natrium-Urin-Ausscheidung abzüglich der Futter-Natriumaufnahme. Zeichnung 1B zeigt den Serumphosphatspiegel. Im Vergleich zu bekannten Wirkstoffen zur Regulierung des Mineralhaushaltes bei Patienten mit eingeschränkter Nierenfunktion führt nur der Wirkstoff gemäß Beispiel 1 gleichzeitig zu einem signifikanten Einfluss auf die Natriumbilanz und den Serumphosphatspiegel.

### Beschreibung Zeichnung 2:

Es ist eine hochaufgelöste transmissionselektronenmikroskopische Aufnahme eines typischen oktaedrischen Maghämit-Kristalles des Stoffes dargestellt, der gemäß Beispiel 1 hergestellt wurde, wobei die längste Ausdehnung des Maghämitkristalls nur 3.5 nm beträgt. Die selektive Elektronenbeugung (SAED=Selected Area Elektron Diffraction) ergibt ein Beugungsmuster, das typisch für Magnetit-Maghämit-Kristalle ist.

### Beschreibung Zeichnung 3

Die Größenverteilung der Kristalle wurde anhand des längsten Durchmessers der Kristalle in transmissionselektronenmikroskopischer Aufnahmen bestimmt. Die Zeichnung 3 A zeigt die Größenverteilung für den Stoff gemäß Beispiel 1 mit einer sehr gleichmäßigen Größenverteilung ohne Anteil an sehr großen Kristallen. Im Vergleich dazu zeigt die Zeichnung 3 B die Größenverteilung gemäß Vergleichsbeispiel 2. Wie ersichtlich, weist ein erheblicher Anteil an Kristallen eine Größe von über 4 nm auf.

### Beispiele

Methode zur Bestimmung der Phosphatbindungskapazität in einer simulierten gastrointestinalen Passage mit Überschuss des anorganischen Phosphates

Es wurde eine 100 mM Lösung aus Natriumdihydrogenphosphat (Sigma-Aldrich) in 0,1 M Salzsäure hergestellt. Die Lösung wurde auf 37°C erwärmt und bei dieser Temperatur gehalten. 40 ml dieser Lösung wurden mit dem Eisen-enthaltenden Phosphatbinder in einer Menge versetzt, dass sich ein molares Verhältnis in der Inkubationslösung von anorganischem Phosphat zu Eisen von 1 : 0,4 einstellt. Der pH-Wert wurde mit Salzsäure auf 1,2 eingestellt. Nach jeweils einer Stunde wurde ein Aliquot von 0,5 ml entnommen und danach der pH-Wert mittels Titrator in den Stufen pH 2,5, 4,5, 7,0, und 7,5 eingestellt. Der Phosphatgehalt wurde mit der Ammonium-Molybdat-Methode photometrisch bei der Extinktion von 880 nm im Filtrat nach Zentrifugation durch einen 3kD CentriPrep® Filter (regenerierte Zellulose) bestimmt. Der freie komplexierbare Eisengehalt des Filtrates wurde mit der Orthophenanthrolin-Methode photometrisch bei der Extinktion von 520 nm bestimmt.

### Vergleichsbeispiel 1

Eine Maghämit-basiertes Phosphatadsorbens wurde gemäß WO 2013/034267 A1 hergestellt. Es wurde 7,55 g Eisen-III-chlorid Hexahydrat (Sigma-Aldrich) in 50 ml bidestilliertem und auf 4°C gekühltem Wasser gelöst (Lösung A). Zu Lösung A wurde 3,2 g Eisen-II-chlorid-Tetrahydrat (Sigma-Aldrich) zugefügt und gelöst (Lösung B). Desweiteren wurde 25 g D-Mannose (Sigma-Aldrich) in bidestilliertem und auf 4°C gekühltem Wasser gelöst (Lösung C). Lösung B und C wurden vereint und 2 min gerührt (Lösung D). Zu Lösung D wurde 100 ml mit 1,5 M NaOH versetztes Wasser (gekühlt auf 4°C) zugefügt und die entstehende Mischung 5 min gerührt bei 4°C, bis ein homogenes Kolloid entsteht (ca. 5 min) und danach auf 60°C erwärmt und 15 min bei 60 °C weitergerührt. Innerhalb von 15 min wurde die Lösung unter Rühren auf Raumtemperatur abgekühlt und mittels Ultrafiltration (10 kD, Spectrum, Hollow Fiber, PES) auf 100 ml reduziert. Die Lösung wurde mit Dialyseschläuchen (12-14 kD Cutoff regenerierte Celullose, Spectra Por) 5 mal gegen 2 Liter bidestilliertes Wasser dialysiert, bis im Filtrat kein Eisen und Chlorid mehr nachweisbar war. Die nach der Dialyse vorhandene kolloidale Lösung in einer Gesamtmenge von 200 ml wurde mit 0,1 g Mannose, 3 g Gummi Arabicum (Acaciabaum Reagent Grade, Sigma) und 3 g Inulin (Sigmaaldrich) versetzt, die gemeinsam in 25 ml bidestilliertem Wasser gelöst worden waren. Diese Dispersion wurde 3 min gerührt und mit 100% Ethanol auf 1 I aufgefüllt. Damit wurden die Nanopartikel ausgefällt und zusätzlich mit 800 ref zentrifugiert. Das Sediment wurde über Nacht bei 60°C getrocknet. Die entstandene Trockensubstanz wurde zu einem Pulver fein gemörsert. Das so entstandene Pulver hatte einen Eisengehalt von 157 mg/g Trockensubstanz bei 2,04% Anteil an zweiwertigem Eisen im Verhältnis zum Gesamteisen. Die Auswertung von 500 Kristallen ergab einen durchschnittlich längsten Durchmesser von 3,4±1,9 nm mit einem Anteil von 90% der Kristalle kleiner 10 nm, jedoch mit einem Anteil von < 20 % der Kristalle zwischen 5 und 10 nm.

| pH | Phosphatbindungskapazität [mg PO₄% / mgFe] | freies Eisen Gewichtsanteil% von Einwaage |
|---|---|---|
| 1,2 | 670 | 26 |
| 2,5 | 790 | 6 |
| 4,5 | 910 | <1 |
| 7 | 950 | <1 |
| 7,5 | 970 | <1 |
| Tabelle 3: Phosphatbindungskapazität bei Inkubation 100 mM PO₄ zu 40 mM Fe für Vergleichsbeispiel 1. | | |

| Größe längster Durchmesser [nm] | Größenverteilung Anteil in % der Gesamtzahl |
|---|---|
| 0,0 - < 0,5 | 0 |
| 0,5 - < 1,0 | 0 |
| 1,1 - < 1,5 | 2 |
| 1,5 - < 2,0 | 8 |
| 2,0 - < 2,5 | 32 |
| 2,5 - < 3,0 | 22 |
| 3,0 - < 3,5 | 12 |
| 3,5 - < 4,0 | 4 |
| 4,0 - < 4,5 | 2 |
| 4,5 - < 5,0 | 1 |
| 5,5 - < 6,0 | 3 |
| 6,0 - < 6,5 | 2 |
| 6,5 - < 7,0 | 3 |
| 7,0 - < 7,5 | 3 |
| 7,5 - < 8,0 | 3 |
| 8,0 - < 8,5 | 1 |
| 8,5 - < 9,0 | 0 |
| 9,0 - < 9,5 | 1 |
| 9,5 - < 10,0 | 0 |
| 10,0 - < 10,5 | 1 |
| Tabelle 2: Größenverteilung der Eisenoxidkristalle basierend auf der Auswertung von 500 Kristallen in der transmissionselektronenmikroskopische Aufnahme der Probe nach Vergleichsbeispiel 1. | |

### Beispiel 1

50 ml Wasser werden in einem Eis-Wasserbad bis zum Temperaturequilibrium gekühlt. Darin werden nacheinander 3,2 g Eisen-(II)-chlorid-Tetrahydrat und 7,55 g Eisen-(III)-chlorid-Hexahydrat gelöst = Lösung 1. In einem weiteren Gefäß werden in 100 ml gleichermaßen gekühlter 1,5 M Natriumhydroxid-Lösung 25 g D-Mannitol und 5 g Inulin gelöst = Lösung 2. Lösung 1 wird zügig in Lösung 2 gegossen und weiter in Eis-Wasserkühlung 15 min gerührt. Dann werden 3 ml 30%ige Wasserstoffperoxidlösung zugeführt, 5 Minuten gerührt und dann unter Rühren auf 60°C erhitzt und weitere 15 min gerührt. Die Probe wird spontan auf Raumtemperatur gekühlt und mittels Dialyse gegen Wasser gereinigt und das Retentat 10 Minuten bei 4500 rpm (Rotorradius 15 cm) zentrifugiert. Der Überstand wird mit 3 g Gummi Arabicum versetzt und die so entstandene Lösung am Rotationsverdampfer eingeengt und danach gefriergetrocknet. Das entstehende rotbraune Pulver hat einen Eisenanteil von 190 mg/g und einen Anteil von zweiwertigem Eisen < 1%. Die mittels TEM nachgewiesene Kristallgröße liegt für über 90% der Kristalle zwischen 0,5 und 4 nm. Das Elektronenbeugungsmuster ergibt ein für Maghämit charakteristisches Beugungsmuster gemäß HKL Klassifikation: 220 Ebene = 0,297 nm; 311 Ebene = 0,254 nm; 400 Ebene = 0,214 nm; 511 Ebene = 0,164 nm; 440 Ebene = 0.151 nm. Die Auswertung von 500 Kristallen ergibt einen durchschnittlich längsten Durchmesser von 3,0±0,6 nm mit einem Anteil von 90 % der Kristalle kleiner 4,5 nm.

| pH | Phosphatbindungskapazität [mg PO₄% / mg Fe] | freies Eisen Gewichtsanteil% von Einwaage |
|---|---|---|
| 1,2 | 1185 | 8 |
| 2,5 | 1502 | <1 |
| 4,5 | 1734 | <1 |
| 7 | 1577 | <1 |
| 7,5 | 1605 | <1 |
| Tabelle 3: Phosphatbindungskapazität bei Inkubation 100 mM PO₄ zu 40 mM Fe für Beispiel 1. | | |

| Größe längster Durchmesser [nm] | Größenverteilung Anteil in % der Gesamtzahl |
|---|---|
| 0,0 - < 0,5 | 0 |
| 0,5 - < 1,0 | 2 |
| 1,1 - < 1,5 | 17 |
| 1,5 - < 2,0 | 34 |
| 2,0 - < 2,5 | 27 |
| 2,5 - < 3,0 | 15 |
| 3,0 - < 3,5 | 3 |
| 3,5 - < 4,0 | 1 |
| 4,0 - < 4,5 | 1 |
| Tabelle 4: Größenverteilung der Eisenoxidkristalle basierend auf der Auswertung von 500 Kristallen in der transmissionselektronenmikroskopische Aufnahme der Probe nach Beispiel 1. | |

### Beispiel 2

Es wurden männliche Ratten der Rasse Sprague Dawley von Charles River mit Gewicht zu Versuchsbeginn von 200 g eingesetzt (n=8) pro nachfolgend aufgezählter Gruppe. Die Tiere erhielten Futter ad libitum Altromin 1324 (Pulverform) in der ersten Versuchswoche (Woche 1) ohne Wirkstoffzusatz (0.7% Phosphor und 0.2% Natrium bezogen auf Gewichtsanteil). Dann für weitere 4 Wochen (Woche 2-5) das o.g. Futter ad libitum mit Wirkstoffzusatz. Am letzten Tag jeder Versuchswoche wurden die Tiere für 24 Stunden (Tag 6 auf Tag 7) einzeln in einem Stoffwechselkäfig gehalten. Kot und Urin wurde gesammelt. Blut wurde nur am letzten Sammeltag gewonnen. Blut und Urinproben wurden untersucht.

Die Gruppen erhielten wie folgt von Woche 2-5 Zusätze je 100 g Futter
Gruppe A Kontrolle - keine Zusätze
Gruppe B Hilfsstoffe - Zugabe von 0,2 g Mannitol und je 0,9 g Inulin und Gummi Arabicum
Gruppe C Beispiel 1 - bezogen auf Eisen als Zusatz zum Futter 250 mg Eisen
Gruppe D Velphoro® - bezogen auf Eisen als Zusatz zum Futter 250 mg Eisen
Gruppe E Fosrenol® - bezogen auf Lanthan als Zusatz zum Futter 250 mg Lanthan
Gruppe F Renvela® - bezogen auf Sevelamer Carbonat 500 mg

Die Ergebnisse stellen sich für die 24 Stunden Sammelperiode am letzten Versuchstag (5 Wochen Gesamtversuch und 4 Wochen Wirkstoff-Fütterung) wie folgt dar:
Ergebnisse der Studie sind in Tabelle 4 dargestellt.

| Tabelle 4 Ergebnisse Studie gemäß Beispiel 2 Datenerhebung Tag 7 Woche 5 | | | | | |
|---|---|---|---|---|---|
| Gruppe A | Gruppe B | Gruppe C | Gruppe D | Gruppe E | Gruppe F |
| Körpergewicht [g] | | | | | |
| 438±29 | 391±33* | 375±31** | 404±28 | 374±42*** | 466±25 |

| Futteraufnahme 24 Stunden [g] | | | | | |
|---|---|---|---|---|---|
| 18,75±4,0 | 23,5±4,6 | 25,3±2,6* | 26,6±4,5 | 21,6±4,7 | 18,4±2,9 |

| Natriumaufnahme 24 Stunden [mmol] | | | | | |
|---|---|---|---|---|---|
| 1,76±0,38 | 2,20±0,43* | 2,37±0,24*** | 2,50±0,42*** | 2,03±0,44 | 1,72±0,27 |

| Natriumausscheidung Urin 24 Stunden [mmol] | | | | | |
|---|---|---|---|---|---|
| 1,64±0,19 | 1,53±0,21 | 1,3±0,36 | 1,43±0,23 | 1,61±0,26 | 1,73±0,14 |

| Natriumbilanz Aufnahme Futter minus Ausscheidung Urin 24 Stunden [mmol] | | | | | |
|---|---|---|---|---|---|
| -0,12±0,38 | -0,58±0,34* | -1,07±0,40*** | -1,15±0,46*** | -0,42±0,44 | 0,01±0,37 |

| Kot Ausscheidung 24 Stunden Gewicht [g] | | | | | |
|---|---|---|---|---|---|
| 14,1±3,0 | 12,9±1,6 | 14,6±3,2 | 17,1±6,2 | 13,1±2,6 | 14,7±3,1 |

| Urin Volumen 24 Stunden [ml] | | | | | |
|---|---|---|---|---|---|
| 24,9±5,5 | 16,7±5,6 | 17,4±6,9 | 16,1±3,6 | 21,3±9,4 | 26±8,3 |

| Serum Natrium [mmol/l] | | | | | |
|---|---|---|---|---|---|
| 141±1,4 | 140±1,5 | 140±1,5 | 141±1,5 | 140±1,0 | 142±2,2 |

| Serum Phosphat [mmol/l] | | | | | |
|---|---|---|---|---|---|
| 2,54±0,40 | 2.42±0.30 | 1,80±0,37*** | 2,41±0,21 | 2,16±0,39 | 2,39±0,4 |

Ein statistischer Vergleich der Analysenwerte der Kontrollgruppe mit den Wirkstoffgruppen erfolgte mit dem Programm Prism 5.0f® mit dem Test One-Way Anova und Dunnetts Postprocessing (* p< 0,05; ** pO.01 ; ***p<0.005).

### Beispiel 3: Reduzierung der renalen Osteodystrophie an dem Tiermodell der urämischen Ratte durch Ko-Fütterung des erfindungsgemäßen Stoffes nach 1

Es wurde eine Urämie an Ratten (Sprague-Dawley, männlich, n=6, Charles-River) erzeugt durch Ko-Fütterung von Adenin (0,3% Zusatz Gewichtsanteil, Futter Altromin C100 ,it 1,2 % Phosphor und 1,2 % Calcium für einen Zeitraum von 10 Wochen). Nach Ablauf der 10-Wochen wurde das Adenin abgesetzt und 3 Tiere erhielten die oben genannte Diät weiterhin für 4 Wochen ohne Wirkstoff (Kontrollgruppe) und die anderen 3 Tiere erhielten dann die oben genannte Diät supplementiert mit 0,125% bezogen auf einen Gewichtsanteil Eisen den erfindungsgemäßen Stoff gemäß Beispiel 1 (Wirkstoffgruppe).

Nach dieser zweiten 4-Wochen-Periode wurden die Tiere getötet, die Oberschenkelknochen entnommen und deren Länge bestimmt (durch rechte und linke Seite insgesamt n=6 Knochen). Es ergab sich ein signifikanter Unterschied in der diaphysären Dicke der Corticalis mittels (Mikro-Computertomographie bestimmt) von 0,42 ± 0.08 mm für die Kontrollgruppe und 0,59 ± 0, 12 mm für die Wirkstoffgruppe.

## Patentansprüche

1. Verfahren zur Herstellung von Maghämit-Kristallen mit einer Kristallgröße im Bereich von 0.5 bis 4 nm und einem Magnetitanteil von kleiner als 5 Gewichtsanteilen von Hundert, wobei sich der Gewichtsanteil auf den Anteil zweiwertiger Eisenionen am Gesamteisen bezieht und das Verfahren die Schritte umfasst:
S1: Herstellen einer wässrigen Natriumhydroxid-Lösung mit einem pH-Wert im Bereich von 10 bis 15, wobei die Natriumhydroxid-Lösung ein oder mehrere Alditole sowie ein oder mehrere Kohlenhydrate enthält und die Temperatur der Natriumhydroxid-Lösung 0 bis 10° Celsius beträgt;
S2: Herstellen einer weiteren wässrigen Lösung, die Chlorid-Ionen, Eisen-II-Salze und Eisen-III-Salze enthält und eine Temperatur von 0 bis 10° Celsius aufweist; und
S3: Herstellen einer Mischung aus der Natriumhydroxid-Lösung und der weiteren Lösung, wobei die Temperatur der Mischung 0 bis 10° Celsius beträgt.

2. Verfahren nach Anspruch 1, bei dem die Natriumhydroxid-Lösung Inulin, Gummi Arabicum und Mannitol enthält.

3. Verfahren nach Anspruch 1, bei dem die im Verfahrensschritt S3 gebildeten Magnetit-Kristalle mittels Zugabe von Wasserstoffperoxid bei Temperaturen über 50°C zu Maghämit-Kristallen oxidiert werden.

## Claims

1. A method for the preparation of maghemite crystals having a crystal size in the range of 0.5 to 4 nm and a magnetite content of less than 5 parts by weight of one hundred, the parts by weight referring to the proportion of divalent iron ions in the total iron and the method comprising the steps:
S1: preparing an aqueous sodium hydroxide solution having a pH value in the range of 10 to 15, the sodium hydroxide solution containing one or more alditols and one or more carbohydrates and the temperature of the sodium hydroxide solution being 0 to 10° Celsius;
S2: preparing a further aqueous solution containing chloride ions, iron(II) salts and iron(III) salts and having a temperature of 0 to 10° Celsius; and
S3: preparing a mixture of the sodium hydroxide solution and the further solution, the temperature of the mixture being 0 to 10° Celsius.

2. The method according to claim 1, in which the sodium hydroxide solution contains inulin, gum arabic and mannitol.

3. The method according to claim 1, in which the magnetite crystals formed in step S3 are oxidized by means of the addition of hydrogen peroxide to maghemite crystals at temperatures above 50°C.

## Revendications

1. Procédé de fabrication de cristaux de maghémite ayant une taille de l'ordre de 0,5 à 4 nm et une proportion de magnétite inférieure à 5 parts en poids pour cent, la part en poids se référant à la part d'ions de fer bivalents dans le fer total et le procédé comprenant les étapes suivantes :
S1 : préparation d'une solution aqueuse d'hydroxyde de sodium ayant un pH de l'ordre de 10 à 15, la solution d'hydroxyde de sodium contenant un ou plusieurs alditols et un ou plusieurs hydrates de carbone et la température de la solution d'hydroxyde de sodium s'élevant à 0 à 10 °C ;
S2 : préparation d'une autre solution aqueuse qui contient des ions de chlorure, des sels de fer II, et des sels de fer III et présente une température de 0 à 10 °C ; et
S3 : préparation d'un mélange composé de la solution d'hydroxyde de sodium et de l'autre solution, la température du mélange s'élevant à 0 à 10 °C.

2. Procédé selon la revendication 1, dans lequel la solution d'hydroxyde de sodium contient de l'inuline, du caoutchouc, de la gomme arabique et du mannitol.

3. Procédé selon la revendication 1, dans lequel, les cristaux de magnétique formés dans l'étape opératoire S3 sont oxydés en cristaux de maghémite par ajout de peroxyde d'hydrogène à des températures supérieures à 50 °C.
